# EUROPEAN PATENT APPLICATION

(11) **EP 2 085 403 A1**
(43) Date of publication of application: **05.08.2009**
(21) Application number: 07829293.5
(22) Date of filing: 05.10.2007
(51) Int. Cl.: C07K 1/113, C07C 319/20, C07C 323/58, C07K 1/08

(54) **PROCESS FOR PRODUCTION OF PEPTIDE THIOESTER**

(30) Priority: 06.10.2006 JP 2006275022
(71) Applicant: TOKAI UNIVERSITY EDUCATIONAL SYSTEM, Shibuya-ku, Tokyo 151-0063 (JP)
(72) Inventor: HOJO, Hironobu, Hiratsuka-shi Kanagawa 259-1292 (JP); NAKAHARA, Yoshiaki, Hiratsuka-shi Kanagawa 259-1207 (JP)
(74) Representative: Polypatent
(86) International application number: PCT/JP2007/069555
(87) International publication number: WO 2008/044628

(57) **Abstract**

An object of the present invention is to provide a method for synthesizing a peptide thioester by using a compound that can be easily obtained within a relatively short time under conditions in which a side reaction is unlikely to occur. In the present invention, a thioester bond is formed by elongating a pepetide chain using N-alkyl cysteine as the C-terminal amino acid according to the Fmoc method, carrying out deprotection, and then causing the peptide bond to undergo N-S transfer to the thiol group of N-alkyl cysteine under weak acidic conditions.

SEQUENCE LISTING

<110>  Tokai University Educational System

<120>  Process for production of peptide thioester

<130>  P22334EP

<140>  EP 07 829 293.5
<141>  2007-10-05

<150>  JP 0275022
<151>  2006-10-06

<160>  17

<170>  PatentIn version 3.3

<210>  1
<211>  10
<212>  PRT
<213>  Artificial

<220>
<223>  synthetic peptide

<400>  1

Glu Val Thr Gly His Arg Trp Leu Lys Gly
1               5                   10

<210>  2
<211>  10
<212>  PRT
<213>  Artificial

<220>
<223>  synthetic peptide

<400>  2

Ala Thr Glu Val Thr Gly His Arg Trp Leu
1               5                   10

<210>  3
<211>  10
<212>  PRT
<213>  Artificial

<220>
<223>  synthetic peptide

<400>  3

Trp Leu Lys Gly Gly Val Val Leu Lys Glu
1               5                   10


<210>  4
<211>  10
<212>  PRT
<213>  Artificial

<220>
<223>  synthetic peptide

<400>  4

Thr Glu Val Thr Gly His Arg Trp Leu Lys
1               5                   10


<210>  5
<211>  11
<212>  PRT
<213>  Artificial

<220>
<223>  synthetic peptide

<400>  5

Glu Val Thr Gly His Arg Trp Leu Lys Gly Cys
1               5                   10


<210>  6
<211>  11
<212>  PRT
<213>  Artificial

<220>
<223>  synthetic peptide

<400>  6

Ala Thr Glu Val Thr Gly His Arg Trp Leu Cys
1               5                   10


<210>  7
<211>  11
<212>  PRT
<213>  Artificial

<220>
<223>  synthetic peptide

<400>  7

Trp Leu Lys Gly Gly Val Val Leu Lys Glu Cys
1               5                   10


<210>  8
<211>  11
<212>  PRT
<213>  Artificial

<220>
<223>  synthetic peptide

<400>  8

Thr Glu Val Thr Gly His Arg Trp Leu Lys Cys
1               5                   10


<210>  9
<211>  25
<212>  PRT
<213>  Artificial

<220>
<223>  synthetic peptide

<400>  9

Gly Ser Lys Ile Leu Leu Thr Cys Ser Leu Asn Asp Ser Ala Thr Glu
1               5                   10                  15


Val Thr Gly His Arg Trp Leu Lys Gly
            20                  25


<210>  10
<211>  15
<212>  PRT
<213>  Artificial

<220>
<223>  synthetic peptide

<400>  10

Asp Ser Ala Thr Glu Val Thr Gly His Arg Trp Leu Lys Gly Cys

1               5                   10                  15


<210>  11
<211>  26
<212>  PRT
<213>  Artificial

<220>
<223>  synthetic peptide

<400>  11

Gly Ser Lys Ile Leu Leu Thr Cys Ser Leu Asn Asp Ser Ala Thr Glu
1               5                   10                  15


Val Thr Gly His Arg Trp Leu Lys Gly Cys
            20                  25


<210>  12
<211>  25
<212>  PRT
<213>  Artificial

<220>
<223>  synthetic peptide

<400>  12

Gly Ser Lys Ile Leu Leu Thr Cys Ser Leu Thr Asp Ser Ala Thr Glu
1               5                   10                  15


Val Thr Gly His Arg Trp Leu Lys Gly
            20                  25


<210>  13
<211>  26
<212>  PRT
<213>  Artificial

<220>
<223>  synthetic peptide

<400>  13

Gly Ser Lys Ile Leu Leu Thr Cys Ser Leu Thr Asp Ser Ala Thr Glu
1               5                   10                  15

Val Thr Gly His Arg Trp Leu Lys Gly Cys
            20                  25

<210>  14
<211>  20
<212>  PRT
<213>  Artificial

<220>
<223>  synthetic peptide

<400>  14

Val Thr Ser Ala Pro Asp Thr Arg Pro Ala Pro Gly Ser Thr Ala Pro
1               5                   10                  15

Pro Ala His Gly
            20

<210>  15
<211>  12
<212>  PRT
<213>  Artificial

<220>
<223>  synthetic peptide

<400>  15

Arg Ser Leu Ala Arg Trp Leu Glu Arg Gln Gly Cys
1               5                   10

<210>  16
<211>  34
<212>  PRT
<213>  Artificial

<220>
<223>  synthetic peptide

<400>  16

Asp Cys His Leu Gln Ala Val Val Leu His Leu Ala Arg Arg Ser Val
1               5                   10                  15

Cys Val His Pro Gln Asn Arg Ser Leu Ala Arg Trp Leu Glu Arg Gln
            20                  25                  30

Gly Cys

<210>  17
<211>  33
<212>  PRT
<213>  Artificial

<220>
<223>  synthetic peptide

<400>  17

Asp Cys His Leu Gln Ala Val Val Leu His Leu Ala Arg Arg Ser Val
1               5                   10                  15

Cys Val His Pro Gln Asn Arg Ser Leu Ala Arg Trp Leu Glu Arg Gln
            20                  25                  30

Gly

## Description

### Technical Field

The present invention relates to a novel method for synthesizing a peptide thioester. More specifically, the present invention relates to a novel method for synthesizing a peptide thioester using an N-alkylcysteine derivative.

### Background Art

Protein chemical synthesis is carried out by repeated condensation of a peptide thioester prepared by solid phase synthetic method. The peptide thioesters that are key substances of this method are generally synthesized by a Boc method (the method using t-butoxy carbonylated amino acid) in consideration of the stability of thioester bond. In recent years, interest concerning post translational protein (such as sugar chains) modification, is increasing. In view of unstability of such modification under acidic conditions, it is desired to prepare a peptide thioester by a Fmoc method which does not use strong acidic conditions. However, thioester bonds are cleaved by piperidine, which is used for Fmoc group removal, so that the conventional Fmoc method is not directly applicable. Accordingly, methods for obtaining a thioester using such Fmoc method have been developed, such as a method using a deprotecting reagent capable of removing a Fmoc group in the presence of a thioester group instead of piperidine and a method for obtaining a thioester by elongating the peptide chain on a sulfonamide linker by the Fmoc method and then carrying out alkylation of the linker and thiolysis. However, all of these methods have both merits and demerits. A general method for thioester synthesis using the Fmoc method has not been established yet.

For example, non-patent documents 1 and 2 report a method that involves elongating a peptide chain by the Fmoc method on a sulfonamide linker, carrying out N-alkylation, so as to unstabilize the peptide bonds, and thus obtaining a peptide thioester via thiolysis. This method applies the fact that an amide bond with an imino group more easily undergoes thiolysis than an amide bond with an amino group. However, the method is that thioesterification may not proceed after peptide chain elongation (non-patent document 3). It has also been reported that this method is that when solid phase synthesis of glycopeptides is carried out with free sugar chain hydroxyl groups, sugar chain hydroxyl groups are alkylated by N-alkylation (non-patent document 4).

Non-patent documents 5 to 8 report methods for preparing a thioester by N-S transfer. The method of non-patent document 5 is that N-S transfer at the final stage is not quantitative. Furthermore, the method of non-patent document 6 is that when a conventional Fmoc method using piperidine is employed, peptide chain release is observed to some extent during peptide chain elongation, and the synthesis of a compound for N-S transfer is complicated. In the methods disclosed in non-patent documents 7 and 8 conducted by the present inventors, a proline derivative is used as a compound for N-S transfer. This method involves converting an amide bond with a secondary amine to a thioester. However, the method lacks practicality such that the synthesis of a proline derivative is complicated and the reaction speed should be increased by unstabilizing amide bonds by irradiation with microwaves, since the transfer proceeds very slowly (taking approximately 1 week).

Furthermore, JP Patent Publication (Kokai) No. 11-217397 A (1999) and non-patent document 9 disclose that a peptide chain can be elongated without cleaving thioester bonds when 1-methyl pyrrolidine or a basic reagent containing hexamethylenimine is used alone or in combination as a reagent for removal of Fmoc group in place of piperidine. However, according to this method, a peptide chain is elongated after thioester bond formation at the C terminal position. Hence, even if another reagent is used for Fmoc group removal, cleavage of thioester bonds still gradually continues and the yield of a target product cannot be increased, as revealed by the research conducted by the present inventors. Moreover, upon introduction of the initial 3 amino acid residues, special condensation conditions are required for suppressing peptide chain release and protocols for automatic peptide synthesis require program modification.
[Non-patent document 1] Shin, Y.; Winans, K. A.; Backes, B. J.; Kent, S. B. H.; Ellman, J. A.; Bertozzi, C. R. J. Am. Chem. Soc., 121, 11684-11689 (1999).
[Non-patent document 2] Ingenito, R.; Bianchi, E.; Fattori, D.; Pessi, A. J. Am. Chem. Soc., 121, 11369-11374 (1999).
[Non-patent document 3] Marcaurelle, L. A., Mizoue, L. S., Wilken, J., Oldham, L. Kent, S. B. H., Handel, T. M. and Bertozzi, C. R. Chem. Eur. J., 7, 1129-1132 (2001).
[Non-patent document 4] Kajihara, Y.; Yoshihara, A.; Hirano, K.; Yamamato, N. Carbohydrate Res., 341, 1333-1340 (2006).
[Non-patent document 5] Kawakami, T.; Sumida, M.; Nakamura, K.; Vorherr, T.; Aimoto, S., Tetrahedron Lett., 46, 8805-8807 (2005).
[Non-patent document 6] Ohta, Y.; Itoh, S; Shigenaga, A,; Shintaku,S.; Fujii, N.; Otaka, A. Org. Lett., 8, 467 (2006).
[Non-patent document 7] Nagaike, et al., Lecture Abstracts, Annual Meeting of Japan Society for Bioscience, Biotechnology, and Agrochemistry, p. 324 (2005).
[Non-patent document 8] Nagaike, F., Onuma, Y., Kanazawa, C., Hojo, H, Ueki, A., Nakahara, Y., Nakahara, Y., Org. Lett., 8, 4465-4468 (2006).
[Non-patent document 9] Li, X.; Kawakami, T.; Aimoto, S. Tetrahedron Lett. 39, 8669-8672 (1998).
[Patent document 1] JP Patent Publication (Kokai) No. 11-217397 A (1999)

### Disclosure of the Invention

### Object to be achieved by the present invention

An object to be achieved by the present invention is to address the above problems of the conventional technology. Specifically, an object of the present invention is to provide a novel method for producing a peptide thioester, by which a target peptide thioester can be synthesized using a compound that can be easily obtained as a reaction reagent within a relatively short time under conditions in which a side reaction is unlikely to occur and to provide an N-alkylcysteine derivative to be used for the production method.

### Means for solving the object

As a result of intensive studies to achieve the above object, the present inventors have discovered that a thioester bond can be formed by elongating a pepetide chain using N-alkyl cysteine as the C-terminal amino acid according to the Fmoc method, carrying out deprotection, and then causing the peptide bond to undergo N-S transfer to the thiol group of N-alkyl cysteine under weak acidic conditions. The present inventors have thus completed the present invention.

The present invention provides a method for producing a peptide thioester, comprising the steps of:
(i) deprotecting Fmoc-NH-resin and then reacting the resultant with Fmoc-(amino acid residue)n-N(R)-CH(CH₂SY)-C(=O)OH (where n is 0 or 1) so as to produce an Fmoc-(amino acid residue)n-N(R)-CH(CH₂SY)-C(=O)NH-resin;
(ii) deprotecting the Fmoc group of the Fmoc-(amino acid residue)n-N(R)-CH(CH₂SY)-C(=O)NH-resin (where n is 0 or 1) obtained in step (i), and if necessary, reacting the resultant with Fmoc-amino acid, so as to produce Fmoc-(amino acid residue)n-N(R)-CH(CH₂SY)-C(=O)NH-resin (where n is 1 or 2), and then repeating Fmoc deprotection and reaction with Fmoc-amino acid, so as to produce X-N(R)-CH(CH₂SY)-C(=O)-NH-resin;
(iii) causing a deprotecting reagent to act on the X-N(R)-CH(CH₂SY)-C(=O)-NH-resin obtained in step (ii), so as to carry out release from the resin and deprotection of a thiol group; and
(iv) causing acidic thiol to act on the compound obtained in step (iii), so as to produce a thioester compound;
(where Fmoc denotes a 9-fluorenyl methoxycarbonyl group, R denotes an alkyl group having carbon number of 1 to 12 or an aralkyl group having carbon number of 7 to 12, Y denotes a protecting group for a thiol group, and X denotes a peptide residue).

Preferably, the deprotecting reagent in step (iii) is trifluoroacetic acid.

Preferably, the acidic thiol in step (iv) is mercaptocarboxylic acid or a mixture of mercaptan and carboxylic acid.

Preferably, the acidic thiol in step (iv) is HSCH₂CH₂COOH (MPA), HSC₆H₄CH₂COOH (MPAA), or a mixture of thiophenol and acetic acid.

Preferably, R is a methyl group, an ethyl group, an isobutyl group, or a benzyl group.

Preferably, Y is a trityl group.

The present invention further provides a compound represented by Z-N(R)-CH(CH₂SY)-C(=O)OH (where Z denotes a hydrogen atom or a 9-fluorenyl methoxycarbonyl group, R denotes an alkyl group having carbon number of 1 to 12 or an aralkyl group having carbon number of 7 to 12, and Y denotes a protecting group for thiol).

The present invention further provides a method for producing the aforementioned compound of the present invention, comprising the steps of:
(i) reacting a compound represented by YSCH₂CH(NH₂)C(=O)OH (where Y is a protecting group for a thiol group) with a compound represented by R¹CHO (where R¹ denotes a hydrogen atom, an alkyl group having carbon number of 1 to 11, or an aryl group having carbon number of 6 to 11), so as to produce a compound reprsented by YSCH₂CH(N=CHR¹)C(=O)OH (where Y and R¹ are as defined above); and
(ii) causing a hydrogenating agent to act on the compound represented by YSCH₂CH(N=CHR¹)C(=O)OH (where Y and R¹ are as defined above) obtained in step (i), so as to produce a compound represented by YSCH₂CH(NHCH₂R¹)C(=O)OH (where Y and R¹ are as defined above), and, if desired, causing 9-fluorenylmethoxycarbonyl-N-hydroxysuccinimide ester to act, so as to protect the amino group with the 9-fluorenylmethoxycarbonyl group.

Preferably, the hydrogenating agent in step (ii) is NaBH₄ or NaBH₃CN.

### Effect of the Invention

The method for producing a peptide thioester accoridng to the present invention is very useful as a method for producing a peptide thioester using the Fmoc method, since the method is free from the problem of the decomposition of thioester bonds by piperidine. The method for producing a peptide thioester according to the present invention is a highly practical method for producing a peptide thioester in that: a cysteine derivative that can be synthesized from a commercial product is used; peptide stability is high during peptide chain elongation; and thioesterification at the final stage proceeds quantitatively and rapidly. Also, the method for producing a peptide thioester of the present invention is a highly practical method compared with conventional thioester synthesis methods because of its low degree of racemization.

### Preferred embodiments for carrying out the invention

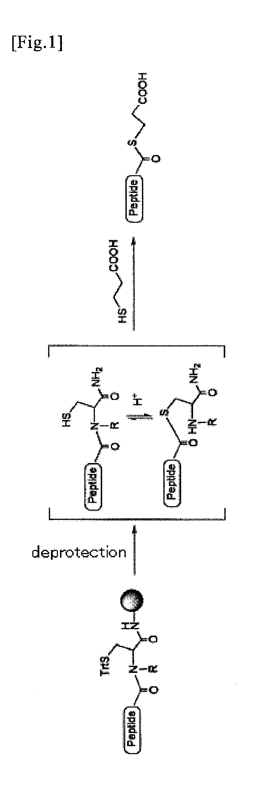

Hereinafter, the present invention will be more specifically described. The method of the present invention comprises elongating a peptide chain according to the Fmoc method using N-alkyl cysteine as the C-terminal amino acid, carrying out deprotection, and then causing a peptide bond to undergo N-S transfer to the thiol group of N-alkyl cysteine under weak acidic conditions, so as to form a thioester bond (Fig. 1).

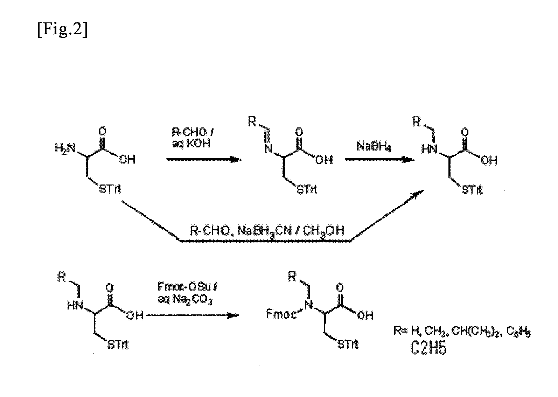

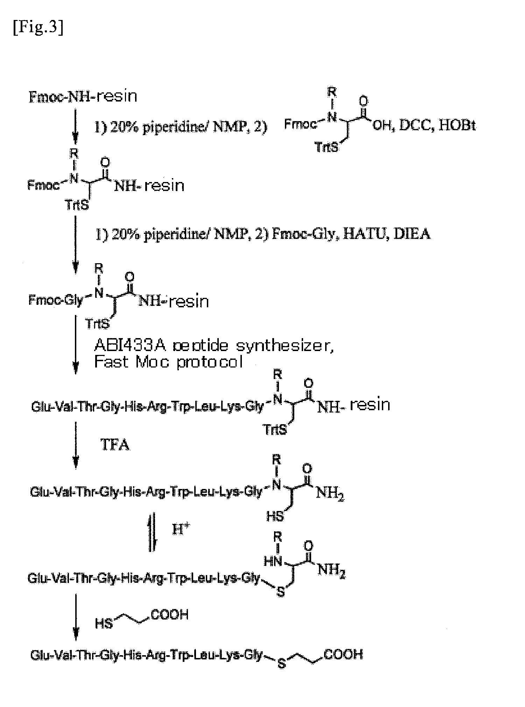

Hereinafter, the present invention is explained with reference to Examples in the description. First, according to the method described in Fig. 2, 3 types of N-alkylcysteine derivative were synthesized. Acetaldehyde, isobutyl aldehyde, or benzaldehyde was reacted with a commercially available Cys(Trt) as a starting material. The thus generated Schiff base was reduced with sodium borohydride, Fmoc protection was carried out, and then the resultant was used for peptide synthesis.

Next, peptide synthesis and thioesterification in a manner described in Fig. 3 were attempted using Glu-Val-Thr-Gly-His-Arg-Trp-Leu-Lys-Gly (SEQ ID NO: 1) as a model peptide. With the use of an Fmoc-CLEAR AMIDE resin as a starting material, an N-alkylcysteine derivative was introduced by a DCC-HOBt method and then Fmoc-Gly was introduced using HATU-DIEA. Thereafter, peptide chain elongation was carried out according to the FastMoc method using Fmoc amino acid by operating of a 433A peptide synthesizer (ABI). Simultaneously with peptide chain release from the resin by the action of Reagent K (TFA) and deprotection, the thiol group of terminal N-alkyl cysteine was also deprotected. The thus obtained crude peptide was left to stand in a 5% 3-mercaptopropionic acid aqueous solution and then the reaction was monitored by HPLC. As a result, the reaction was completed within approximately 20 hours.

Furthermore, to examine the degree of racemization when an optically active amino acid was used as the C-terminus, Fmoc-N-Et-Cys(Trt) was introduced and then a peptide chain was synthesized using Leu, Lys, or Glu as the C-terminal amino acid. Thioesterification was carried out using 5% 3-mercaptopropionic acid. The resultant was compared with a separately prepared product whose C-terminus was D-amino acid in terms of the elution position of HPLC, so that the degree of racemization was examined. As a result, the degree of racemization when the Lys had been used as the C-terminus was approximately 2.5% and the degree of racemization when Leu had been used as the C-terminus was approxiamtely 7%.

As described above, based on the method of the present invention, a model peptide comprising 10 residues and having Gly at the C-terminus was synthesized. Then the thioesterification thereof was attempted. It was revealed that a target peptide thioester can be obtained within approximately 1 day almost without side reaction. Also, thioesterification of a peptide having Leu, Lys, or Glu as the C-terminal amino aicd proceeded. As a result, the degree of racemization of the C-terminal amino acid was 7% or less. The degree of racemization was lower than that of conventional thioester synthesis methods. Based on the above results, it was demonstrated that the method of the present invention is a practical peptide thioester synthesis method.

The method for producing a peptide thioester according to the present invention comprises the following steps (i) to (iv). The steps (i) to (iv) are as described below.

Step (i) comprises deprotecting Fmoc-NH-resin and then reacting the resultant with Fmoc-(amino acid residue)n-N(R)-CH(CH₂SY)-C(=O)OH (where n is 0 or 1), so as to produce Fmoc-(amino acid residue)n-N(R)-CH(CH₂SY)-C(=O)NH-resin. In step (i), Fmoc-amino acid residue-N(R)-CH(CH₂SY)-C(=O)NH-resin can be produced by reacting NH₂-resin with Fmoc-amino acid residue-N(R)-CH(CH₂SY)-C(=O)OH instead of Fmoc-N(R)-CH(CH₂Y)-C(=O)OH. When the rate of introduction of Fmoc-amino acid into NH(R)-CH(CH₂SY)-C(=O)NH-resin is low, the introduction rate can be improved by the method.

In the present invention, R denotes a C1-12 alkyl group or a C7-12 aralkyl group. Examples of such alkyl group include a methyl group, an ethyl group, a propyl group, a butyl group, a pentyl group, and a hexyl group, which may be a linear, branched, or cyclic hydrocarbon group. Examples of such aralkyl group include a benzyl group. R is prferably a methyl group, an ethyl group, an isobutyl group, or a benzyl group.

When R is hydrogen, specifically when cysteine bonds to an Fmoc group, a thioester exchange reaction barely proceeds as described later in comparative example 1. It is important that R is an alkyl group or an aralkyl group.

In the present invention, Y denotes a protecting group for a thiol group. Examples of such protecting group for a thiol group include a trityl group, an acetamide group, a benzyl group, a methylbenzyl group, a 4-methoxybenzyl group, a 3-nitro-2-pyridine sulphenyl group, an ethylmercapto group, a tertiary butylmercapto group, and a tertiary butyl group. Y is preferably a trityl group.

Step (i) can be carried out by treating an Fmoc-CLEAR amide resin with piperidine/N-methylpyrrolidone (NMP) so as to deprotect the Fmoc group and then reacting the resin with Fmoc-N(R)-CH(CH₂SY)-C(=O)OH, HOBt/NMP and N-dicyclohexylcarbodiimide (DCC)/NMP, for example. The reaction can be carried out for a predetermined time at appropriate temperatures (e.g., 40°C to 60°C).

Step (ii) for producing X-N(R)-CH(CH₂SY)-C(=O)-NH-resin comprises deprotecting the Fmoc group of the Fmoc-(amino acid residue)n-N(R)-CH(CH₂SY)-C(=O)NH-resin (where n is 0 or 1) obtained in step (i), reacting the resultant with Fmoc-amino acid if necessary, so as to produce Fmoc-(amino acid residue)n-N(R)-CH(CH₂SY)-C(=O)NH-resin (where n is 1 or 2), and then repeating Fmoc deprotection and the reaction with Fmoc-amino acid. In addition, in step (i), when Fmoc-(amino acid residue)n-N(R)-CH(CH₂SY)-C(=O)NH-resin is produced by reacting the resultant with Fmoc-(amino acid residue)n-N(R)-CH(CH₂SY)-C(=O)OH (where n is 1), Fmoc-amino acid may be caused to react or not to react in step (ii).

In step (ii), specifically, the resin is shaken for 5 minutes in Ac₂O-DIEA/NMP after washing the reaction product in step (i) with NMP. After washing with NMP and Fmoc group removal with piperidine/NMP, reaction is carried out by addition of a solution prepared by dissolving Fmoc-Gly, hexafluorophosphoric acid O-(7-azabenzotriazol-1-yl)-N,N,N',N'-tetramethyluronium (HATU), and N,N-diisopropylethylamine (DIEA) in NMP. The reaction can proceed at an appropriate temperature (e.g., 40°C to 60°C) for a predetermined time. Subsequently, the reaction product is washed with NMP and then Fmoc group deprotection and reaction with Fmoc-amino acid are repeated by a FastMoc method, for example, using a general peptide synthesizer, so that the peptide chain can be elongated.

Step (iii) comprises causing a deprotecting reagent (e.g., trifluoroacetic acid) to act on X-N(R)-CH(CH₂SY)-C(=O)-NH-resin obtained in step (ii), so as to carry out the release from the resin and deprotection of thiol groups. Specifically, Reagent K is added to the product (resin) in step (ii) and then a reaction can proceed at room temperature. Here, the term "Reagent K" refers to a reagent of the following mixture; TFA : H₂O : thioanisole : ethanedithiol : phenol = 82.5 : 5 : 5 : 2.5 : 5.

Step (iv) comprises causing acidic thiol to act on the compound obtained in step (iii), so as to produce a thioester compound. Specific examples of acidic thiol to be used herein include mercaptocarboxylic acid and a mixture of mercaptan and carboxylic acid. More preferably, HSCH₂CH₂COOH (MPA), HSC₆H₄CH₂COOH (MPAA), or a mixture of thiophenol and acetic acid can be used.

In step (iv), TFA is removed from the product in step (iii) by nitrogen stream and then ether is added, so as to cause precipitation. The precipitate is washed with ether and then dried. A crude peptide is extracted with an acetonitrile aqueous solution containing TFA, diluted with a 3-mercaptopropionic acid aqueous solution or an aqueous solution such as acetonitrile of acidic thiol (e.g., 4-mercaptophenyl acetic acid), and then allowed to stand for several hours to dozens of hours. Thus, a peptide thioester compound represented by X-S-CH₂CH₂COOH or X-S-C₆H₄CH₂COOH can be obtained.

The present invention further relates to a compound represented by Z-N(R)-CH(CH₂SY)-C(=O)OH (wherein Z denotes a hydrogen atom or a 9-fluorenylmethoxycarbonyl group, R denotes a C1-12 alkyl group or a C7-12 aralkyl group, and Y denotes a protecting group for thiol). Detailed description concerning groups represented by R and Y are as described above. Hereinafter, a method for producing the above compound is explained.

First, a compound represented by YSCH₂CH(N=CHR¹)C(=O)OH (where Y and R¹ are as defined below) is produced by reacting a compound represented by YSCH₂CH(NH₂)C(=O)OH (where Y is a protecting group for a thiol group) (specifically, cysteine with a protected thiol group) with R¹CHO (where R¹ denotes a hydrogen atom or a C1-11 alkyl group or a C6-11 aryl group). Here, an alkyl group denoted by R¹ is preferably a C1-9 alkyl group, and further preferably a C1-6 alkyl group. Examples of such alkyl group include a methyl group, an ethyl group, a propyl group, a butyl group, a pentyl group, and a hexyl group, which may be linear, branched, or cyclic hydrocarbon. R¹ is particularly preferably a methyl group, an isopropyl group, or a phenyl group. Reaction with a compound represented by R¹CHO can be carried out by dissolving cysteine with a protected thiol group in water containing ethanol and potassium hydroxide, adding a compound represented by R¹CHO to the solution, and then stirring at an appropriate temperature (e.g., room temperature) for predetermined time.

Subsequently, a compound represented by Z-N(R)-CH(CH₂SY)-C(=O)OH (where Z denotes a hydrogen atom or a 9-fluorenylmethoxycarbonyl group, R denotes a C1-12 alkyl group or a C7-12 aralkyl group, and Y denotes a protecting group for a thiol group) can be produced by causing a hydrogenating agent (e.g, NaBH₄ or NaBH₃CN) to act on a compound represented by YSCH₂CH(N=CHR¹)C(=O)OH (wherein Y and R¹ are as defined above) so as to produce a compound represented by YSCH₂CH(NHCH₂R¹)C(=O)OH (wherein Y and R¹ are as defined above) and if desired causing 9-fluorenylmethoxycarbonyl-N-hydroxysuccinimide ester (hereinafter, Fmoc-OSu) to act, so as to protect the amino group with an Fmoc group. When a hydrogenating agent is caused to act, the reaction may be carried out in the presence of a base such as sodium hydroxide. Specifically, the reaction can be carrid out by adding a hydrogenating agent (e.g., NaBH₄ or NaBH₃CN) to a compound represented by YSCH₂CH(N=CHR¹)C(=O)OH and then stirring the solution at an appropriate temperature. When NaBH₄ is used, NaBH₄ is preferably dissolved in a sodium hydroxide aqueous solution and then used. When NaBH₃CN is used, a base is not particularly required. Furthermore, when Fmoc-OSu is caused to act, so as to protect the amino group with an Fmoc group, the amino group can be protected with the Fmoc group by causing Fmoc-OSu to act in the presence of sodium carbonate in an appropriate solvent (e.g., 1,2-dimethoxyethane).

The present invention will be explained more specifically with reference to the following examples, but the present invention is not limited by the examples.

### Examples

### Example 1

### (1): N-Ethyl-S-trityl-L-cysteine (1): Method using NaBH₄ as a hydrogenating agent

S-Trityl-L-cysteine (1.0 g, 2.8 mmol) was dissolved in water (3.0 ml) containing ethanol (7.0 ml) and KOH (150 mg, 2.8 mmol). Acetaldehyde (0.20 ml, 3.6 mmol) was added to the solution. The solution was stirred for 1 hour at room temperature, NaBH₄ (0.10 g, 2.8 mmol) dissolved in water (2.0 ml) containing 2 droplets of 1M NaOH was added, and then the solution was stirred at room temperature for 15 minutes and then at 60°C for 10 minutes. After the temperature returned to room temperature, pH was adjusted at 4 using 1M HCl and then a target product was precipitated. After this, the precipitate was allowed to stand at 4°C for 1 hour and then filtered. After washing with a small volume of distilled water, the resultant was dried at 50°C under reduced pressure overnight. The thus obtained powders were purified by silica gel chromatography (chloroform : methanol = 4 : 1; containing of 1% acetic acid), so that the target product (0.34 g, 0.87 mmol, 31%) was obtained.

### (2) N-Ethyl-S-trityl-L-cysteine (2): Method using NaBH₃CN as a hydrogenating agent

S-Trityl-L-cysteine (1.0 g, 2.8 mmol) and NaBH₃CN (0.17 g, 2.8 mmol) were suspended in methanol (28 ml) and then acetaldehyde (0.19 ml, 3.3 mmol) was added thereto. The thus obtained solution was stirred at room temperature for 1 hour and then the solvent was distilled off under reduced pressure. The residue was dissolved in 1-butanol, washed with distilled water, and then washed with saturated saline. The resultant was dried with the use of sodium sulfate. The residue obtained by distilling off the solvent was purified by silica gel chromatography (chloroform : methanol = 4 : 1; containing of 1% acetic acid), so that a target product (0.52 g, 1.3 mmol, 48%) was obtained.
[α]_{D} +30.8° (c 1.1 in CHCl₃). R_{f} 0.27 (9:1 CHCl₃-CH₃OH containing 1%AcOH). ¹H-NMR (CDCl₃): δ3.03 (brt, 1H, J=6.1 Hz, αH), 2.81 (m, 1H, βH), 2.71 (m, 1H, βH), 2.56 (m, 2H, -CH₂CH₃), 1.04 (brt, 3H, J= 7.3 Hz, CH₃-) HRFABMS: Calcd for C₂₄H₂₅NO₂S · Na: 414.15037, found: m/z 414.14576.

### Example 2: N-(9-Fluorenylmethoxycarbonyl)-N-ethyl-S-trityl-L-cysteine

N-Ethyl-S-trityl-L-cysteine (220 mg, 0.56 mmol) was dissolved in a 10% sodium carbonate aqueous solution (3 ml) and 1,2-dimethoxyethane (1.5 ml). Fmoc-OSu (300 mg, 0.89 mmol) dissolved in 1,2-dimethoxyethane (1.5 ml) was added to the solution. The solution was stirred at room temperature overnight. After the precipitate was filtered, the filtrate was neutralized with 1 M HCl. A target product was extracted with ethyl acetate and then dried using anhydrous sodium sulfate. After the solvent was removed, the thus obtained residue was purified by silica gel chromatography (toluene : ethyl acetate = 2 : 1; containing of 1% acetic acid). Thus, the target product (280 mg, 0.46 mmol, 82%) was obtained.
[α]_{D} -39.6° (c 1.1 in CHCl₃). R_{f} 0.31 (2:1 Toluene-ethyl acetate containing 1% AcOH). ¹H-NMR (CDCl₃): δ4.51-4.33 (m, 2H, Fmoc -CH₂-),4.19 (brt, J=6.6Hz, 0.6H, Fmoc -CH-), 4.09 (m, 0.4H, Fmoc -CH-), 3.39 (m, 0.4H, -CH₂CH₃), 3.28 (m, 0.6H, -CH₂CH₃), 3.11 (m, dd, J=5.8, 8.8 Hz, 0.6H, αH), 2.98-2.85 (m, 0.4H; αH, 1.2H; βH x2), 2.74-2.59 (m, 1H; -CH₂CH₃, 0.4H; βH), 2.39 (dd, J=9.3, 13.7 Hz, 0.4H, βH), 0.91-0.85 (m, 3H, -CH₃). HRFABMS: Calcd for C₃₉H₃₅NO₄SNa: 636.21845, found: m/z 636.21236.

### Example 3: N-Isobutyl-S-trityl-L-cysteine

S-Trityl-L-cysteine (1.0 g, 2.8 mmol) was dissolved in water (3.0 ml) containing ethanol (7.0 ml) and KOH (150 mg, 2.8 mmol) and then isobutyl aldehyde (0.30 ml, 3.3 mmol) was added. After the solution was stirred for 1 hour at room temperature, NaBH₄ (0.13 g, 3.4 mmol) dissolved in water (2.0 ml) containing 2 droplets of 1M NaOH was added. The solution was stirred at room temperature for 15 minutes and then at 60°C for 10 minutes. After the temperature returned to room temperature, pH was adjusted at 4 using 1M HCl, so as to precipitate a target product. After the precipitate was allowed to stand at 4°C for 1 hour, the precipitate was filtered, washed with a small volume of distilled water, and then dried at 50°C under reduced pressure overnight. The thus obtained powders were purified by silica gel chromatography (chloroform : methanol = 7 : 1; containing of 1% acetic acid), so that the target product (0.79 g, 1.9 mmol, 67%) was obtained.
[α]_{D} +26.1° (c 1.0 in CHCl₃-CH₃OH 1:1). R_{f} 0.34 (9:1 CHCl₃-CH₃OH containing 1% AcOH). ¹H-NMR (CDCl₃-CD₃OD):δ2.96-2.88 (m, 2H, αH, βH), 2.69 (dd, 1H, J=7.8, 12.7 Hz, βH), 2.40-2.32 (m, 2H, CH₂CH(CH₃)₂), 1.83 (m, 1H, -CH(CH₃)₂), 0.90 (m,6H, -CH₃). HRFABMS: Calcd for C₂₆H₂₉NO₂S · Na: 442.18167, found: m/z 442.18614.

### Example 4: N-(9-Fluorenylmethoxycarbonyl)-N-isobutyl-S-trityl-L-cysteine

N-Isobutyl-S-trityl-L-cysteine (870 mg, 2.1 mmol) was dissolved in 10% sodium carbonate aqueous solution (10 ml) and 1,2-dimethoxyethane (5 ml). Fmoc-OSu (1.0 g, 3.0 mmol) dissolved in 1,2-dimethoxyethane (10 ml) was added and then the solution was stirred at room temperature overnight. After the precipitate was filtered, the filtrate was neutralized using 1 M HCl. A target product was extracted with ethyl acetate and then dried using anhydrous sodium sulfate. After the solvent was removed, the thus obtained residue was purified by silica gel chromatography (toluene : ethyl acetate = 3 : 1; containing of 1% acetic acid). Thus the target product (1.0 g, 1.6 mmol, 75%) was obtained.
[α]_{D} -56.1° (c 1.0 in CHCl₃). R_{f} 0.22 (4:1 toluene-ethyl acetate containing 1% AcOH). ¹H-NMR (CDCl₃): δ4.47 (m, 1.3H, Fmoc -CH₂-), 4.36 (m, 0.7H, Fmoc -CH₂-), 4.14 (m, 0.7H, Fmoc -CH-), 4.08 (m, 0.3H, Fmoc -CH-), 3.17 (dd, 0.3H, 8.8, 13.7 Hz, -CH₂CH(CH₃)₂), 3.05 (dd, 0.7H, 4.9, 12.2 Hz, βH), 2.91-2.75 (m, αH, 0.7H,βH, 0.7H, -CH₂CH(CH₃)₂), 2.70 (m, 0.3H, βH), 2.37-2.32 (m, 0.3H,βH, 0.3H, -CH₂CH(CH₃)₂), 2.22 (dd, 0.7H, 6.8, 14.1 Hz, -CH₂CH(CH₃)₂), 1.45 (m, 0.3H, -CH(CH₃)₂), 1.35 (m, 0.7H, -CH(CH₃)₂), 0.77 (m, 1.8H, -CH₃), 0.65 (d, 2.1H, 6.8 Hz, -CH₃), 0.58 (d, 2.1H, 6.3 Hz, -CH₃). HRFABMS: Calcd for C₄₁H₃₉NO₄S · Na: 664.24975, found: m/z 664.24466.

### Example 5: N-Benzyl-S-trityl-L-cysteine

S-Trityl-L-cysteine (1.0 g, 2.8 mmol) was dissolved in water (3.0 ml) containing ethanol (7.0 ml) and KOH (150 mg, 2.8 mmol) and then benzaldehyde (0.30 ml, 3.0 mmol) was added. After the solution was stirred for 1 hour at room temperature, NaBH₄ (0.11 g, 2.9 mmol) dissolved in water (1.0 ml) containing two droplets of 1M NaOH was added. The solution was stirred at room temperature for 15 minutes and then at 60°C for 10 minutes. After the temperature returned to room temperature, pH was adjusted at 4 using 1M HCl, so as to precipitate a target product. After the precipitate was allowed to stand at 4°C for 1 hour, the precipitate was filtered, washed with a small volume of distilled water, and then dried at 50°C under reduced pressure overnight. The thus obtained residue was purified by silica gel chromatography (chloroform:methanol =13:1; containing of 1% acetic acid), so that the target product (0.26 g, 0.60 mmol, 22%) was obtained.
[α]_{D} 29.8° (c 1.0 in CHCl₃). R_{f} 0.31 (13:1 chloroform-methanol containing 1% AcOH). ¹H-NMR (CDCl₃): δ3.63 (brs, 2H, -CH₂-Ph), 3.02 (brt, 1H, J=5.9 Hz, αH), 2.73 (m, 2H, βH). HRFABMS: Calcd for C₂₉H₂₇NO₂S · Na: 476.16602, found: m/z 476.16271.

### Example 6: N-(9-Fluorenylmethoxycarbonyl)-N-benzyl-S-trityl-L-cysteine

N-Benzyl-S-trityl-L-cysteine (220 mg, 0.49 mmol) was dissolved in a 10% sodium carbonate aqueous solution (3.5 ml) and then Fmoc-OSu (0.25 g, 0.74 mmol) dissolved in 1,2-dimethoxyethane (1.0 ml) was added. The solution was stirred at room temperature overnight. After the precipitate was filtered, the filtrate was neutralized using 1 M HCl. A target product was extracted with ethyl acetate and then dried using anhydrous sodium sulfate. After the solvent was removed, the thus obtained residue was purified by silica gel chromatography (toluene : ethyl acetate = 5 : 1; containing of 1% acetic acid). Thus, the target product (180 mg, 0.27 mmol, 54%) was obtained.
[α]_{D} -45.4° (c 1.0 in CHCl₃). R_{f} 0.25 (5:1 toluene-ethyl acetate containing 1% AcOH). ¹H-NMR (CDCl₃): δ4.63 (d, 0.3H, J=15.6Hz, -CH₂-Ph), 4.44 (d, 0.7H, J=16.1 Hz, -CH₂-Ph), 4.12 (m, 1H, Fmoc -CH-), 3.91 (d, 0.3H, J=15.1 Hz, -CH₂-Ph), 3.89 (d, 0.7H, J=16.1 Hz, -CH₂-Ph), 3.40 (m, 1H, αH), 2.95 (dd, 0.7H, J=5.8, 13.7 Hz, βH), 2.85 (d, 0.7H, J=8.3, 13.2 Hz, βH), 2.60 (m, 0.3H, βH), 2.45 (m, 0.3H, βH). HRFABMS: Calcd for C₄₄H₃₇NO₄S · Na: 698.23410, found: m/z 698.23063.

### Example 7: N-Methyl-S-trityl-L-cysteine

S-Trityl-L-cysteine (2.0 g, 5.5 mmol) was dissolved in water (6.0 ml) containing ethanol (14 ml) and KOH (300 mg, 5.4 mmol) and then formaldehyde (36-38% aqueous solution, 0.54 ml, 6.6 mmol) was added. The solution was stirred for 1 hour at room temperature and then NaBH₄ (0.25 g, 6.6 mmol) dissolved in water (4.0 ml) containing 4 droplets of 1M NaOH was added. The solution was stirred at room temperature for 15 minutes and then at 60°C for 10 minutes. After the temperature returned to room temperature, pH was adjusted at 4 using 1M HCl, so as to precipitate a target product. After the precipitate was allowed to stand at 4°C for 1 hour, the precipitate was filtered. The resultant was washed with a small volume of distilled water and then dried at 50°C under reduced pressure overnight. The thus obtained powders were purified by silica gel chromatography (chloroform : methanol = 4:1; containing of 1% acetic acid). The resultant was further purified using an ODS column and an acetonitrile aqueous solution containing 0.1% TFA. Thus, the target product (670 mg, 1.77 mmol, 32%) was obtained.
[α]_{D} -9.7° (c 0.5 in CHCl₃). R_{f} 0.27 (6:1 CHCl₃-CH₃OH containing 1% AcOH). ¹H-NMR (CDCL₃): δ 2.96 (m, 1H, αH), 2.77 (m, 1H, βH), 2.59 (m, 1H, βH), 2.03 (bs, 3H, -C*H*₃). HRFABMS: Calcd for C₂₃H₂₃NNaO₂S: 400.1347, found: m/z 400.1781.

### Example 8: N-(9-Fluorenylmethoxycarbonyl)-N-methyl-S-trityl-L-cysteine

N-Methyl-S-trityl-L-cysteine (46 mg, 0.12 mmol) was dissolved in a 10% sodium carbonate aqueous solution (1.0 ml) and 1,2-dimethoxyethane (0.3 ml) and then Fmoc-OSu (82 mg, 0.24 mmol) dissolved in 1,2-dimethoxyethane (0.6 ml) was added. The solution was stirred at room temperature overnight. After the precipitate was filtered, the filtrate was neutralized with 1 M HCl. A target product was extracted with ethyl acetate and then dried using anhydrous sodium sulfate. After the solvent was removed, the thus obtained residue was purified by silica gel chromatography (toluene : ethyl acetate = 1 : 1; containing of 1% acetic acid). Thus, the target product (54 mg, 90 µmol, 74%) was obtained.
[α]_{D} -25.9° (c 1.1 in CHCl₃).
R_{f} 0.22 (2:1 Toluene-ethyl acetate containing of 1% AcOH).
¹H-NMR (CDCl₃): δ4.43-4.33 (m, 2H, Fmoc -C*H*₂-), 4.24 (m, 0.6H, Fmoc -C*H*-), 4.17 (m, 0.4H, Fmoc -C*H*-), 3.97 (dd, 0.6 H, J=4.9, 10.2 Hz, αH), 3.83 (m, 0.4 H, αH), 2.85 (dd, 0.6 H, J=4.9, 13.1 Hz, βH), 2.71 (s, 1.8 H, -C*H*₃), 2.68 (s, 1.2 H, -C*H*₃), 2.60 (dd, 0.4 H, J= 4.9, 13.1 Hz, βH), 2.41 (dd, 0.4H, J=10.7, 13.1 Hz, βH). HRFABMS: Calcd for C₃₈H₃₃NNaO₄S: 622.2028, found: m/z 622.2021.

**Table 1**

| Synthesis yield (%) | | | |
|---|---|---|---|
| | R | 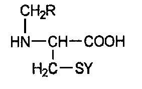 | 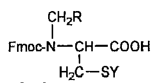 Y: trityl |
| Examples 7 and 8 | H | 32 | 74 |
| Examples 1 and 2 | CH₃ | 31 | 82 |
| Examples 3 and 4 | (CH₃)₂CH₂ | 67 | 75 |
| Examples 5 and 6 | C₆H₅ | 22 | 54 |

### Example 9: N-(9-Fluorenylmethoxycarbonyl)-alanyl-N-ethyl-S-trityl-L-cysteine

Fmoc-alanine 1-hydrate (0.23 g, 0.7 mmol) and HATU (0.27 g, 0.7 mmol) were dissolved in DMF (1 ml) and then DIEA (0.12 ml, 0.7 mmol) was added. The thus obtained solution was stirred at room temperature for 2 minutes. The solution was added to a solution of N-Et-Cys(Trt) (0.20 g, 0.53 mmol) and DIEA (0.092 ml, 0.53 mmol) dissolved in DMF (0.50 ml), followed by 1 hour of reaction at room temperature. After DMF was distilled off under reduced pressure, the residue was dissolved in ethyl acetate. After washing with 1 M hydrochloric acid and saturated saline, the resultant was dried using anhydrous sodium sulfate. After sodium sulfate was filtered, the solution was concentrated. The residue was purified by silica gel chromatography (chloroform : methanol = 19 : 1; containing of 1% acetic acid) and then purified with bio-beads SX-3 (toluene : ethyl acetate = 3:1). Thus, the target product (0.24 g, 0.35 mmol, 67%) was obtained.
[α]_{D} -11.5° (c 1.0 in CHCl₃).
R_{f} 0.29 (19:1 Chloroform-methanol containing 1% AcOH).
¹H-NMR (CDCl₃): δ4.56 (m, 1H, AlaαH), 4.45-4.14 (m, 3H, Fmoc -C*H*-, -C*H*₂-), 1.33-1.29 (m, 3H, Ala -C*H*₃), 1.02-0.96 (m, 3H, -CH₂-C*H*₃).
HRFABMS: Calcd for C₄₂H₄0N₂NaO₅S, 707.2556, found, m/z 707.2421.

### Example 9: Example of synthesis of peptide thioester containing glycine as C-terminal residue using N-alkylcysteine derivative:

### (9-1) Synthesis of Glu-Val-Thr-Gly-His-Arg-Trp-Leu-Lys-Gly-SCH₂CH₂COOH using N-methyl cysteine as C-terminal amino acid

Fmoc-CLEAR amide resin (65 mg, 0.03 mmol) was treated with 20% piperidine/NMP for 5 minutes and the reaction was repeated with fresh reagent for 15 minutes. A solution prepared through the reaction of Fmoc-N-Me-Cys(Trt) (36 mg, 0.06 mmol), 1 M HOBt/NMP (0.09 ml), and 1 M DCC/NMP (0.09 ml) for 30 minutes was added to the resin, followed by 1 hour of reaction at 50°C. After washing with NMP, the resin was shaken in 10% Ac₂O-5% DIEA/NMP for 5 minutes. After washing with NMP and Fmoc removal with 20% piperidine/NMP, a solution of Fmoc-Gly (89 mg, 0.3 mmol), HATU (108 mg, 0.28 mmol), and DIEA (0.10 ml, 0.60 mmol) dissolved in NMP was added, followed by 1 hour of reaction at 50°C. After washing with NMP, peptide chain elongation was carried out according to the FastMoc method using an ABI 433A peptide synthesizer. Thus, Glu(OBu*^{t}*)-Val-Thr(Bu*^{t}*)-Gly-His(Trt)-Arg(Pbf)-Trp(Boc)-Leu-Lys(Boc)-Gly-N-Et-Cy s(Trt)-NH-resin (129 mg) was obtained. Reagent K (0.4 ml) was added to a portion (10 mg) of the resin, followed by 2 hours of shaking at room temperature. TFA was removed by nitrogen stream and then ether was added, so as to cause precipitation. The precipitate was washed three times with ether and then the resultant was dried under reduced pressure. A crude peptide was extracted with 15% acetonitrile aqueous solution (0.2 ml) containing 0.1% TFA. The resultant was diluted with a 5% aqueous solution of 3-mercaptopropionic acid (MPA) (2.0 ml) and then the solution was allowed to stand overnight. The crude peptide was purified by HPLC, so that Glu-Val-Thr-Gly-His-Arg-Trp-Leu-Lys-Gly-SCH₂CH₂COOH (0.98 mg, 0.77 µmol, 33%) was obtained.
MALDI-TOF MS: found, m/z 1270.3 (M+H)⁺: calcd. for (M+H)⁺, 1270.6.
Amino acid analysis: Thr_{0.84}Glu_{0.93}Gly₂Val_{0.90}Leu_{1.10} Lys_{1.00}His_{0.93}Arg_{0.87}.

### (9-2) Synthesis of Glu-Val-Thr-Gly-His-Arg-Trp-Leu-Lys-Gly-SCH₂CH₂COOH using N-ethyl cysteine as the C-terminal amino acid

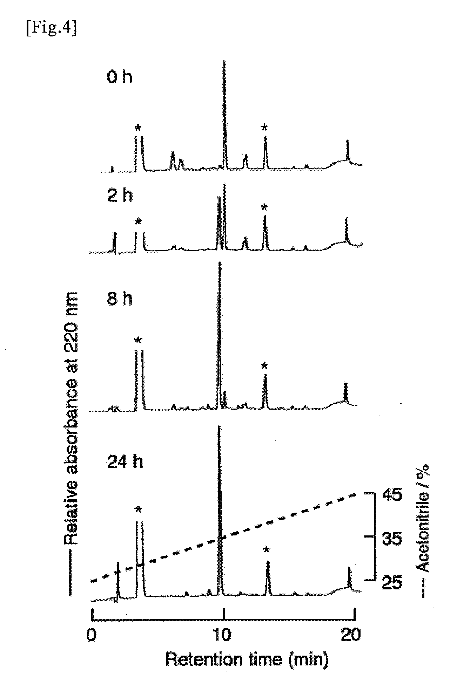

Fmoc-CLEAR amide resin (220 mg, 0.1 mmol) was treated with 20% piperidine/NMP for 5 minutes and the reaction was repeated with fresh reagent for 15 minutes. A solution prepared through 30 minutes of reaction of Fmoc-N-Et-Cys (Trt) (120 mg, 0.2 mmol), 1 M HOBt/NMP (0.3 ml), and 1 M DCC/NMP (0.3 ml) was added to the resin, followed by 1 hour of reaction at 50°C. After washing with NMP, the resin was shaken in 10% Ac₂O-5% DIEA/NMP for 5 minutes. After washing with NMP and Fmoc removal with 20% piperidine/NMP, a solution of Fmoc-Gly (300 mg, 1.0 mmol), HATU (360 mg, 0.95 mmol), and DIEA (0.35 ml, 2.0mmol) dissolved in NMP (1.5 ml) was added, followed by 1 hour of reaction at 50°C. After washing with NMP, peptide chain elongation was carried out according to the FastMoc method using an ABI 433A peptide synthesizer. Thus, Glu(OBu^{t})-Val-Thr(Bu^{t})-Gly-His(Trt)-Arg(Pbf)-Trp(Boc)-Leu-Lys(Boc)-Gly-N-Et-Cy s(Trt)-NH-resin (430 mg) was obtained. Reagent K (0.4 ml) was added to a portion (10 mg) of the resin, followed by 2 hours of shaking at room temperature. TFA was removed by nitrogen stream and then ether was added, so as to cause precipitation. The precipitate was washed 3 times with ether and then dried under reduced pressure. A crude peptide was extracted with a 15% acetonitrile aqueous solution (0.2 ml) containing 0.1% TFA and then diluted with a 5% aqueous solution of mercaptopropionic acid (MPA) (2.0 ml). The resultant was allowed to stand overnight. The crude peptide was purified by HPLC. Thus, Glu-Val-Thr-Gly-His-Arg-Trp-Leu-Lys-Gly-SCH₂CH₂COOH (1.0 mg, 0.81 µmol, 34%) was obtained. In addition, the course of the reaction by HPLC is shown in Fig. 4.
MALDI-TOF MS: found, m/z 1270.8 (M+H)⁺: calcd. for (M+H)⁺, 1270.6.
Amino acid analysis: Thr_{0.95}Glu_{0.87}Gly₂Val_{0.94}Leu_{1.03} Lys_{1.02}His_{0.98}Arg_{0.92}.

In Fig. 4, the peak of RT = 10 min at 0 h represents Glu-Val-Thr-Gly-His-Arg-Trp-Leu-Lys-Gly-N-ethyl-cysteine-NH₂ and the peaks of RT = 10 min that appeared at 8 h and 24 h represent Glu-Val-Thr-Gly-His-Arg-Trp-Leu-Lys-Gly-SCH₂CH₂COOH. In addition, the peak at 4 min is thought to represent MPA and the peak at 12 min is thought to represent the MPA dimer.

### (9-3) Synthesis of Glu-Val-Thr-Gly-His-Arg-Trp-Leu-Lys-Gly-SCH₂CH₂COOH using N-isobutyl cysteine as the C-terminal amino acid

Fmoc-CLEAR amide resin (220 mg, 0.1 mmol) was treated with 20% piperidine/NMP for 5 minutes and the reaction was repeated with fresh reagent for 15 minutes. A solution prepared through 30 minutes of reaction of Fmoc-N-iBu-Cys (Trt) (130 mg, 0.2 mmol), 1 M HOBt/NMP (0.3 ml), and 1 M DCC/NMP (0.3 ml) was added to the resin, followed by 1 hour of reaction at 50°C. After washing with NMP, the resin was shaken in 10% Ac₂O-5% DIEA/NMP for 5 minutes. After washing with NMP, Fmoc removal was carried out using 20% piperidine/NMP. A solution of Fmoc-Gly (300 mg, 1.0 mmol), HATU (360 mg, 0.95 mmol), and DIEA (0.35 ml, 2.0 mmol) dissolved in NMP (1.5 ml) was added, followed by 1 hour of reaction at 50°C. After washing with NMP, peptide chain elongation was carried out according to the FastMoc method using an ABI 433A peptide synthesizer. Thus, Glu(OBu^{t})-Val-Thr(Bu^{t})-Gly-His(Trt)-Arg(Pbf)-Trp(Boc)-Leu-Lys(Boc)-Gly-N-iBu-C ys(Trt)-NH-resin (390 mg) was obtained. Reagent K (0.4 ml) was added to a portion (10 mg) of the resin and then the resultant was shaken at room temperature for 2 hours. TFA was removed by nitrogen stream and then ether was added, so as to cause precipitation. After washing 3 times with ether, the precipitate was dried under reduced pressure. A crude peptide was extracted with 15% acetonitrile aqueous solution (0.2 ml) containing 0.1% TFA, diluted with a 5% aquesous solution of mercaptopropionic acid (MPA) (2.0 ml), and then allowed to stand overnight. The crude peptide was purified by HPLC, so that Glu-Val-Thr-Gly-His-Arg-Trp-Leu-Lys-Gly-SCH₂CH₂COOH (0.85 mg, 0.67 µmol, 28%) was obtained.
MALDI-TOF MS: found, m/z 1270.9 (M+H)⁺: calcd. for (M+H)⁺, 1270.6.
Amino acid analysis: Thr_{0.96}Glu_{0.94}Gly₂Val_{0.93}Leu_{1.02} Lys_{1.02}His_{0.95} Arg_{0.94}.

### (9-4) Synthesis of Glu-Val-Thr-Gly-His-Arg-Trp-Leu-Lys-Gly-SCH₂CH₂COOH using N-benzyl cysteine as C-terminal amino acid

Fmoc-CLEAR amide resin (110 mg, 0.05 mmol) was treated with 20% piperidine/NMP for 5 minutes and the reaction was repeated with fresh reagent for 15 minutes. A solution prepared through 30 minutes of reaction of Fmoc-N-Bn-Cys (Trt) (68 mg, 0.1 mmol), 1 M HOBt/NMP (0.15 ml), and 1 M DCC/NMP (0.15 ml) was added to the resin, followed by 1 hour of reaction at 50°C. After washing with NMP, the resin was shaken in 10% Ac₂O-5%DIEA/NMP for 5 minutes. After washing with NMP and Fmoc removal with 20% piperidine/NMP, a solution of Fmoc-Gly (150 mg, 0.5 mmol), HATU (180 mg, 0.47 mmol), and DIEA (0.18 ml, 1.0mmol) dissolved in NMP (0.8 ml) was added, followed by 1 hour of reaction at 50°C. After washing with NMP, peptide chain elongation was carried out according to the FastMoc method using an ABI 433A peptide synthesizer. Thus, Glu(OBu^{t})-Val-Thr(Bu^{t})-Gly-His(Trt)-Arg(Pbf)-Trp(Boc)-Leu-Lys(Boc)-Gly-N-Bn-C ys(Trt)-NH-resin (180 mg) was obtained. Reagent K (0.4 ml) was added to a portion (10 mg) of the resin and then the solution was shaken at room temperature for 2 hours. TFA was removed by nitrogen stream and then ether was added, so as to cause precipitation. After washing 3 times with ether, the precipitate was dried under reduced presssure. A crude peptide was extracted with 15% acetonitrile aqueous solution (0.2 ml) containing 0.1% TFA, diluted with a 5% aqueous solution of mercaptopropionic acid (MPA) (2.0 ml), and then allowed to stand overnight. The crude peptide was purified by HPLC so that Glu-Val-Thr-Gly-His-Arg-Trp-Leu-Lys-Gly-SCH₂CH₂COOH (0.60 mg, 0.47 µmol, 17%) was obtained.
MALDI-TOF MS: found, m/z 1270.6 (M+H)⁺: calcd. for (M+H)⁺, 1270.6.
Amino acid analysis: Thr_{0.78}Glu_{0.97}Gly₂Val_{0.79}Leu_{1.01}Lys_{0.91}His_{0.87}Arg_{0.82}.

### Comparative example 1: Synthesis of

### Glu-Val-Thr-Gly-His-Arg-Trp-Leu-Lys-Gly-SCH₂CH₂COOH using cysteine as C-terminal amino acid

Peptide chain elongation was carried out according to the FastMoc method using an Fmoc-CLEAR amide resin (110 mg, 0.05 mmol) as the starting material and an ABI 433A peptide synthesizer. Thus, Glu(OBu^{t})-Val-Thr(Bu^{t})-Gly-His(Trt)-Arg(Pbf)-Trp(Boc)-Leu-Lys(Boc)-Gly-Cys(Trt) -NH-resin (209 mg) was obtained. Reagent K (0.4 ml) was added to a portion (10 mg) of the resin and then the solution was shaken at room temperature for 2 hours. TFA was removed by nitrogen stream and then ether was added, so as to cause precipitation. After washing 3 times with ether, the precipitate was dried under reduced pressure. A crude peptide was extracted with 15% acetonitrile aqueous solution (0.2 ml) containing 0.1 % TFA, diluted with a 5% aqueous solution of mercaptopropionic acid (MPA) (2.0 ml), and then allowed to stand overnight. The crude peptide was purified by HPLC, so that Glu-Val-Thr-Gly-His-Arg-Trp-Leu-Lys-Gly-SCH₂CH₂COOH (24 µg, 0.045 µmol, 1.9%) was obtained.
MALDI-TOF MS: found, m/z 1270.8 (M+H)⁺: calcd. for (M+H)⁺, 1270.6.
Amino acid analysis: Thr_{0.84}Glu_{0.89}Gly₂Val_{0.85}Leu_{0.98}Lys_{0.78}His_{0.77}Arg_{0.93}.

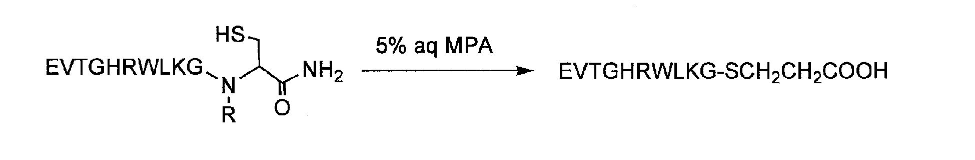

**Table 2**

| | Type of R | Synthesis yield (%) |
|---|---|---|
| Comparative example 1 | H | 1.9 |
| Example | CH₃ | 33 |
| Example | C₂H₅ | 34 |
| Example | CH₂CH(CH₃)₂ | 28 |
| Example | CH₂C₆H₅ | 17 |

| | | |
|---|---|---|
| R: Type of N-R cysteine used | | |

### Example 10: Synthesis of Glu-Val-Thr-Gly-His-Arg-Trp-Leu-Lys-Gly-SC₆H₅ using N-ethyl cysteine as C-terminal amino acid

Reagent K (0.4 ml) was added to a portion (10 mg) of Glu(OBu^{t})-Val-Thr(Bu^{t})-Gly-His(Trt)-Arg(Pbf)-Trp(Boc)-Leu-Lys(Boc)-Gly-N-Et-Cy s(Trt)-NH-resin (430 mg) synthesized in (9-2). The solution was shaken at room temperature for 2 hours. TFA was removed by nitrogen stream and then ether was added, so as to cause precipitation. After washing 3 times with ether, the precipitate was dried under reduced pressure. A crude peptide was extracted with 15% acetonitrile aqueous solution (0.2 ml) containing 0.1% TFA and then diluted with 30% acetonitrile water (1.7 ml). Acetic acid (100 µl) and thiophenol (40 µl) were added to the solution and then the reaction solution was shaken at room temperature for 24 hours. The reaction solution was subjected to purification by HPLC so that Glu-Val-Thr-Gly-His-Arg-Trp-Leu-Lys-Gly-SC₆H₅ (1.0 mg, 0.80 µmol, 35%) was obtained.
MALDI-TOF MS: found, m/z 1274.9 (M+H)⁺: calcd. for (M+H)⁺, 1274.7.
Amino acid analysis: Thr_{0.98}Glu_{0.92}Gly₂Val_{0.90}Leu_{0.99}Lys_{0.98}His_{0.93}Arg_{1.02}.

### Example 11: Example of synthesis of peptide thioester using N-ethyl cysteine derivative, in which C-terminal residue is a residue other than glycine:

### (11-1) Synthesis of Ala-Thr-Glu-Val-Thr-Gly-His-Arg-Trp-Leu-SCH₂CH₂COOH

Fmoc-N-Et-Cys(Trt)-NH-resin (0.03 mmol) 20% was treated with piperidine/NMP for 5 minutes and the reaction was repeated with fresh reagent for15 minutes. A solution of Fmoc-Leu (180 mg, 0.5 mmol), HATU (180 mg, 0.47 mmol), and DIEA (0.23 ml, 1.3 mmol) dissolved in NMP (0.8 ml) was added to the resin, followed by 1 hour of reaction at 50°C. The resin was washed with NMP and then the reaction of the resin with the above Fmoc-Leu was repeated twice. After washing with NMP, peptide chain elongation was carried out according to the FastMoc method using an ABI 433A peptide synthesizer. Thus, Ala-Thr(Bu^{t})-Glu(OBu^{t})-Val-Thr(Bu^{t})-Gly-His(Trt)-Arg(Pbf)-Trp(Boc)-Leu-N-Et-Cys (Trt)-NH-resin (184 mg) was obtained. Reagent K (0.4 ml) was added to a portion (10 mg) of the resin and then the solution was shaken at room temperature for 2 hours. TFA was removed by nitrogen stream and then ether was added, so as to cause precipitation. After washing 3 times with ether, the precipitate was dried under reduced pressure. A crude peptide was extracted with a 15% acetonitrile aqueous solution (0.2 ml) containing 0.1%TFA, diluted with a 5% 3-mercaptopropionic acid (MPA) aqueous solution (2.0 ml), and then allowed to stand over 2 nights. The crude peptide was purified by HPLC, so that Ala-Thr-Glu-Val-Thr-Gly-His-Arg-Trp-Leu-SCH₂CH₂COOH (0.15 mg, 0.12 µmol, 7.0%) was obtained. Furthermore, the degree of racemization was found to be approximately 6.8%.
Amino acid analysis: Thr_{1.81}Glu_{0.93}Gly₁Ala_{0.96}Val_{0.94}Leu_{0.93}His_{0.95}Arg_{0.91}.
MALDI-TOF MS: found, m/z (M+H)⁺: 1258.1, calcd. for (M+H)⁺ 1257.6.

### (11-2) Synthesis of Trp-Leu-Lys-Gly-Gly-Val-Val-Leu-Lys-Glu-SCH₂CH₂COOH

Fmoc-N-Et-Cys(Trt)-NH-resin (0.03 mmol) was treated with 20% piperidine/NMP for 5 minutes and the reaction was repeated with fresh reagent for 15 minutes. A solution of Fmoc-Glu(OBu^{t}) (210 mg, 0.5 mmol), HATU (180 mg, 0.47 mmol), and DIEA (0.23 ml, 1.3 mmol) dissolved in NMP (0.8 ml) was added to the resin, followed by 1 hour of reaction at 50°C. After the resin was washed with NMP, the reaction of the resin with the above Fmoc- Glu(OBu^{t}) was repeated twice. After washing with NMP, peptide chain elongation was carried out according to the FastMoc method using an ABI 433A peptide synthesizer. Thus, Trp(Boc)-Leu-Lys(Boc)-Gly-Gly-Val-Val-Leu-Lys(Boc)-Glu(OBu^{t})-N-Et-Cys(Trt)-N H-resin (90 mg) was obtained. Reagent K (0.4 ml) was added to a portion (10 mg) of the resin and then the solution was shaken at room temperature for 2 hours. TFA was removed by nitrogen stream and then ether was added, so as to cause precipitation. After washing 3 times with ether, the precipitate was dried under reduced pressure. A crude peptide was extracted with a 15% acetonitrile aqueous solution (0.2 ml) containing of 0.1%TFA, diluted with a 5% 3-mercaptopropionic acid (MPA) aqueous solution (2.0 ml), and then allowed to stand over 2 nights. The crude peptide was purified by HPLC, so that Trp-Leu-Lys-Gly-Gly-Val-Val-Leu-Lys-Glu-SCH₂CH₂COOH (0.21mg, 0.18 mmol, 5.3%) was obtained.
Amino acid analysis: Glu_{0.91}Gly₂Val_{1.13}Leu_{1.90}Lys_{2.01}
MALDI-TOF MS: found, m/z (M+H)⁺: 1216.7, calcd. for (M+H)⁺ 1216.7.

### (11-3) Thr-Glu-Val-Thr-Gly-His-Arg-Trp-Leu-Lys-SCH₂CH₂COOH

Fmoc-N-Et-Cys(Trt)-NH-resin (0.03 mmol) was treated with 20% piperidine/NMP for 5 minutes and the reaction was repeated with fresh reagent for 15 minutes. A solution of Fmoc-Lys(Boc) (0.23 g, 0.5 mmol), HATU (180 mg, 0.47 mmol), and DIEA (0.23 ml, 1.3 mmol) dissolved in NMP (0.8 ml) was added to the resin, followed by 1 hour of reaction at 50°C. After the resin was washed with NMP, the reaction of the resin with the above Fmoc-Lys(Boc) was repeated twice. After washing with NMP, peptide chain elongation was carried out according to the FastMoc method using an ABI 433A peptide synthesizer. Thus, Thr(Bu^{t})-Glu(OBu^{t})-Val-Thr(Bu^{t})-Gly-His(Trt)-Arg(Pbf)-Trp(Boc)-Leu-Lys(Boc)-NEt-Cys(Trt)-NH-resin (101 mg) was obtained. Reagent K (0.4 ml) was added to a portion (10 mg) of the resin and then the solution was shaken at room temperature for 2 hours. TFA was removed by nitrogen stream and then ether was added, so as to cause precipitation. After washing 3 times with ether, the precipitate was dried under reduced pressure. A crude peptide was extracted with a 15% acetonitrile aqueous solution (0.2 ml) containing of 0.1% TFA, diluted with a 5% 3-mercaptopropionic acid (MPA) aqueous solution (2.0 ml), and then allowed to stand over 3 nights. The crude peptide was purified by HPLC so that Thr-Glu-Val-Thr-Gly-His-Arg-Trp-Leu-Lys-SCH₂CH₂COOH (0.17 mg, 0.13 µmol, 4.4%) was obtained. Furthermore, the degree of racemization was found to be approximately 2.5%.
Amino acid analysis: Thr_{1.81}Glu_{0.82}Gly₁Val_{0.93}Leu_{0.93}Lys_{1.02}His_{0.99}Arg_{0.92}.
MALDI-TOF MS: found, m/z (M+H)⁺: 1315.0, calcd. for (M+H)⁺ 1314.7.

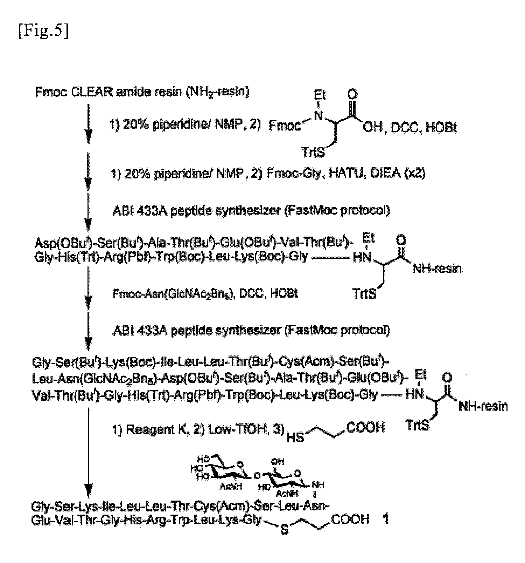

### Example 12-1: Synthesis of N-linked glycopeptide thioester [emmprin (34-58): Gly-Ser-Lys-Ile-Leu-Leu-Thr-Cys(Acm)-Ser-Leu-Asn(GlcNAc₂)-Asp-Ser-Ala-Thr-Gl u-Val-Thr-Gly-His-Arg-Trp-Leu-Lys-Gly-SCH₂CH₂COOH] (Fig. 5)

Fmoc-CLEAR amide resin (220 mg, 0.1 mmol) was treated with 20% piperidine/NMP for 5 minutes and the reaction was repeated with fresh reagent for 15 minutes. A solution prepared through 30 minutes of reaction of Fmoc-N-Et-Cys(Trt) (123 mg, 0.2 mmol), 1 M HOBt/NMP (0.25 ml), and 1 M DCC/NMP (0.25 ml) was added to the resin, followed by 1 hour of reaction. After washing with NMP, the resin was shaken in 10% Ac₂O-5% DIEA/NMP for 5 minutes. After washing with NMP, the resultant was treated with 20% piperidine/NMP for 5 minutes and the reaction was repeated with fresh reagent for 15 minutes. Fmoc-Gly (150 mg, 0.5 mmol), HATU (180 mg, 0.48 mmol), and DIEA (0.17 ml, 1 mmol) were added and then the reaction was carried out at 50°C for 1 hour. The same amounts of Fmoc-Gly, HATU, and DIEA were added again and then the reaction was carried out at 50°C for 1 hour. On the thus obtained resin, peptide chain elongation was carried out according to the FastMoc method using a peptide synthesizer. Thus, Asp(OBu*^{t}*)-Ser(Bu*^{t}*)-Ala-Thr(Bu*^{t}*)-Glu(OBu*^{t}*)-Val-Thr(Bu*^{t}*)-Gly-His(Trt)-Arg(Pbf)-Trp (Boc)-Leu-Lys(Boc)-Gly-N-Et-Cys(Trt)-NH-resin was obtained. A solution prepared by stirring Fmoc-Asn (GlcNAc₂Bn₅) (120 mg, 0.1 mmol), 1 M HOBt/NMP (0.12 ml), and 1 M DCC/NMP (0.12 ml) at room temperature for 30 minutes was added to a half amount (0.05 mmol) of the thus obtained resin, followed by 1 hour of reaction at 50°C. The remaining amino acid sequences were also introduced using an automatic synthesizer, so that Gly-Ser(Bu*^{t}*)-Lys(Boc)-Ile-Leu-Leu-Thr(Bu*^{t}*)-Cys(Acm)-Ser(Bu*^{t}*)-Leu-Asn(GlcNAc₂B n₅)-Asp(OBu*^{t}*)-Ser(Bu*^{t}*)-Ala-Thr(Bu*^{t}*)-Glu(OBu*^{t}*)-Val-Thr(Bu*^{t}*)-Gly-His(Trt)-Arg(Pbf) -Trp(Boc)-Leu-Lys(Boc)-Gly-N-Et-Cys(Trt)-NH-resin (356 mg) was obtained. For deprotection of peptide chain, Reagent K (3.5 ml) was added to a portion (200 mg) of the product and then the solution was shaken at room temperature for 2 hours. TFA was removed by nitrogen stream and then ether was added, so as to cause precipitation. After washing 3 times with ether, the precipitate was drired under reduced pressure. For deprotection of sugar chain portions, a crude peptide was further treated with Low-TfOH [TfOH/TFA/DMS/m-cresol (1:5:3:1, 4.0 ml)] at -15°C for 2 hours and then ether was added, so as to cause precipitation. After washing 3 times with ether, the precipitate was dried under reduced pressure. The crude peptide was dissolved in a 10% acetonitrile aqueous solution (30 ml) containing of 5% mercaptopropionic acid (MPA), the solution was allowed to stand at room temperature for 2 days, and then the resultant was purified by HPLC. Thus, emmprin (34-58)thioester Gly-Ser-Lys-Ile-Leu-Leu-Thr-Cys(Acm)-Ser-Leu-Asn(GlcNAc₂)-Asp-Ser-Ala-Thr-Gl u-Val-Thr-Gly-His-Arg-Trp-Leu-Lys-Gly-SCH₂CH₂COOH **1** (2.3 µmol, 8.2%) was obtained.
MALDI-TOF MS: found, m/z 3251.8 (M+H)⁺: calcd. for (M+H)⁺, 3251.6.
Amino acid analysis: Asp_{1.94}Thr_{2.42}Ser_{2.47}Glu_{0.94}Gly₃
Ala_{1.11}Val_{1.49}Ile_{0.86}Leu₃.₇₈Lys_{1.95}His_{0.98}Arg_{1.03}.

In addition, according to the method disclosed in JP Patent Publication (Kokai) No. 11-217397 A (1999), a similar reaction was carried out using a reagent for Fmoc removal (1-methyl pyrrolidine (25 ml), hexamethylenimine (2 ml), 1-hydroxybenzotriazole (2 g), and a mixed solvent (volume ratio (1/1)) of 1-methyl-2-pyrrolidinone(1-methyl-2-pyrrolidone)/dimethyl sulfoxide (total 100 ml)). The resulting yield was 1.8%.

Reference: Hojo, H. ; Haginoya, E. ; Matsumoto, Y. ; Nakahara, Y. ; Nabeshima, K. ; Toole, B. P. ; Watanabe, Y. Tetrahedron Lett. 2003, 44, 2961-2964.

### Example 12-2: Synthesis of o-linked glycopeptide thioester (emmprin (34-58): Gly-Ser-Lys-Ile-Leu-Leu-Thr-Cys(Acm)-Ser-Leu-Thr[(Gal-GlcNAc)₂-GalNAc]-Asp-S er-Ala-Thr-Glu-Val-Thr-Gly-His-Arg-Trp-Leu-Lys-Gly-SCH₂CH₂COOH 3

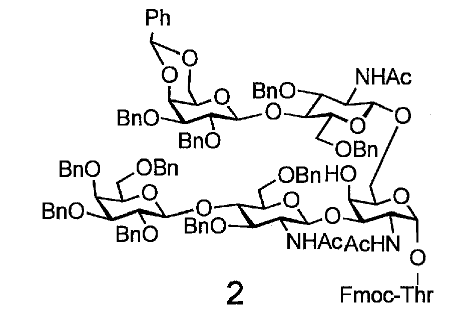

A solution prepared by stirring compound **2** (83 mg, 37 µmol), 1 M HOBt/NMP (0.1 ml), and 1 M DCC/NMP (0.1 ml) at room temperature for 30 minutes was added to 25 µmol of the resin of Asp(OBu*^{t}*)-Ser(Bu*^{t}*)-Ala-Thr(Bu*^{t}*)-Glu(OBu*^{t}*)-Val-Thr(Bu*^{t}*)-Gly-His(Trt)-Arg(Pbf)-Trp (Boc)-Leu-Lys(Boc)-Gly-N-Et-Cys(Trt)-NH-resin obtained upon synthesis of compound 1, followed by 8 hours of stirring at 50°C. The remaining amino acid sequences were elongated by hand using an NMP solution of Fmoc-amino acid (0.1 mmol), DCC (0.15 mmol), and HOBt (0.15 mmol). A portion (85 mg) of the thus obtained Fmoc-Gly-Ser(Bu*^{t}*)-Lys(Boc)-Ile-Leu-Leu-Thr(Bu*^{t}*)-Cys(Acm)-Ser(Bu*^{t}*)-Leu-Thr*-As p(OBu*^{t}*)-Ser(Bu*^{t}*)-Ala-Thr(Bu*^{t}*)-Glu(OBu*^{t}*)-Val-Thr(Bu*^{t}*)-Gly-His(Trt)-Arg(Pbf)-Trp(B oc)-Leu-Lys(Boc)-Gly-N-Et-Cys(Trt)-NH-resin (171 mg) was treated with 20% piperidine/NMP. Reagent K (1.5 ml) was added to the obtained resin and then the solution was shaken at room temperature for 2 hours. TFA was removed by nitrogen stream and then ether was added, so as to cause precipitation. After washing 3 times with ether, the precipitate was dried under reduced pressure. A crude peptide was further treated with Low-TfOH [TfOH/TFA/DMS/m-cresol (1:5:3:1, 0.6 ml)] at -15°C for 2 hours. Ether was added to cause precipitation. After washing 3 times with ether, the precipitate was dried under reduced pressure. The crude peptide was dissolved in water (10 ml) containing 5% MPA and then allowed to stand at room temperature for 3 days. Subseqeuntly the crude peptide was purified by HPLC, so that emmprin (34-58)thioester Gly-Ser-Lys-Ile-Leu-Leu-Thr-Cys(Acm)-Ser-Leu-Thr[(Gal-GlcNAc)₂-GalNAc]-Asp-S er-Ala-Thr-Glu-Val-Thr-Gly-His-Arg-Trp-Leu-Lys-Gly-SCH₂CH₂COOH **3** (0.7 µmol, 5.5%) was obtained.
MALDI-TOF MS: found, m/z 3765.8 (M+H)⁺: calcd. for (M+H)⁺, 3764.8. Amino acid analysis: Asp_{0.97}Thr_{3.33}Ser_{2.43}Glu_{0.94}Gly₃Ala_{1.15}Val_{1.39}Ile_{0.89}Leu_{3.80}
Lys_{2.04}His_{1.00}Arg_{1.00}.

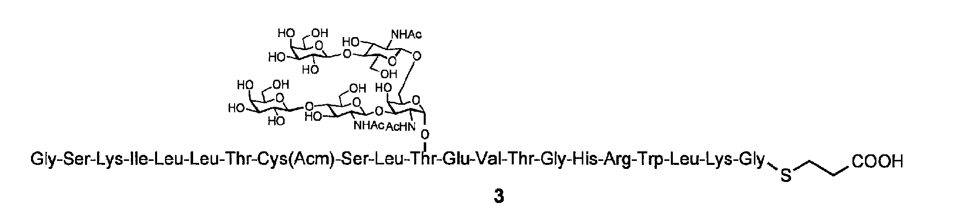

In addition, when the same peptide thioester (however, the C-terminus was thioester amide) was synthesized according to the method disclosed in JP Patent Publication (Kokai) No. 11-217397 A (1999), the resulting yield was 2.2%.

Reference : Ueki, A.; Nakahara, Y.; Hojo, H.; Nakahara, Y. Tetrahedron 2007, 63, 2170-2181.

### Example 12-3: Synthesis of mucin peptide

### Ac-Val-Thr(GalGalNAc)-Ser-Ala-Pro-Asp-Thr-Arg-Pro-Ala-Pro-Gly-Ser-Thr(GalGal NAc)-Ala-Pro-Pro-Ala-His-Gly-SCH₂CH₂COOH 5 having two sugar chains within the moleucle

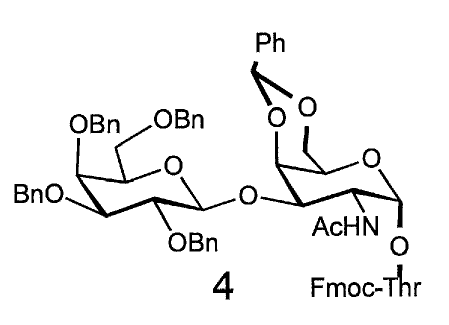

Fmoc-N(Et)-Cys(Trt)-OH (307 mg, 0.5 mmol) was condensed at 50°C using CLEAR Amide resin (0.25 mmol, 544 mg) as the starting material, 1 M DCC (750 µl), and 1 M HOBt (750 µl). After Fmoc deprotection using piperidine, Fmoc-Gly (372 mg, 1.25 mmol) was subjected to condensation reaction at 50°C using HATU (475 mg, 1.25 mmol) and DIEA (327 µl, 1.88 mmol). After 30 minutes, DIEA (109 µl, 0.63 mmol) was added and then another 30 minutes of reaction was carried out. Fmoc-Gly was caused to undergo double coupling. A peptide chain was elongated to a position before the sugar unit on the thus obtained resin using an ABI 433A peptide synthesizer. A portion (0.1 mmol) of the resin was extracted and then subjected to 1 hour of reaction at 50°C with compound **4** (231 mg, 0.2 mmol) using 0.45 M HBTU-HOBt/DMF (422 µl, 0.19 µmol), and DIEA (69.7 ml, 0.4 mmol). Furthermore, the peptide chain was elongated to the next sugar unit using a peptide synthesizer. Fmoc-Thr(GalGalNAc) (231 mg, 0.2 mmol) was reacted using 0.45 M HBTU-HOBt/DMF (422 µl, 0.19 µmol) and DIEA (69.7 ml, 0.4 mmol) at 50°C for 1 hour. Finally, Fmoc-Val (67.8 mg, 0.2 mmol) was condensed using 1 M DCC (300 µl) and 1 M HOBt (300 µl), so that a peptide having the N-terminus protected with an Fmoc group was obtained. A half amount of the resin was subjected to Fmoc deprotection and acetylation using Ac₂O/DIEA/NMP. The thus obtained resin was dried and then the total amount (352 mg) thereof was subjected to 1 hour of reaction with Reagent K (5 ml). The reaction solution was removed by nitrogen gas. Diethyl ether was added to cause peptide precipitation. This procedure was repeated 3 times, so that the precipitate was vacuum dried. The thus obtained peptide was treated with Low-TfOH [TfOH/TFA/DMS/m-cresol (1:5:3:1, 4.0 ml)] at -15°C for 2 hours.

Cooled diethyl ether was added to cause peptide precipitation. This procedure was repeated 3 times, so that the precipitate was vacuum dried. After drying, the resultant was dissolved in 2 ml of distilled water. Five (5)% mercaptopropionic acid was then added, followed by 2 days of reaction. The product was purified by HPLC, so that target glycopeptide thioester 5 was obtained. The amount of the thus obtained peptide was 18.9 mg (6.9 mmol, 14%).
MALDI-TOF MS: found: m/z 2747.74 (M+H)⁺, calcd for: 2747.20 (M+H)⁺.
Amino acid analysis: Asp_{1.00}Thr_{2.73}Ser_{1.93}Pro_{5.05}Gly_{2.00} Ala_{3.88}Val_{0.96}His_{1.00}Arg_{0.96}.

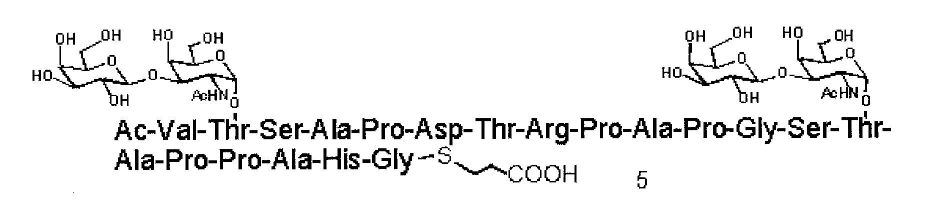

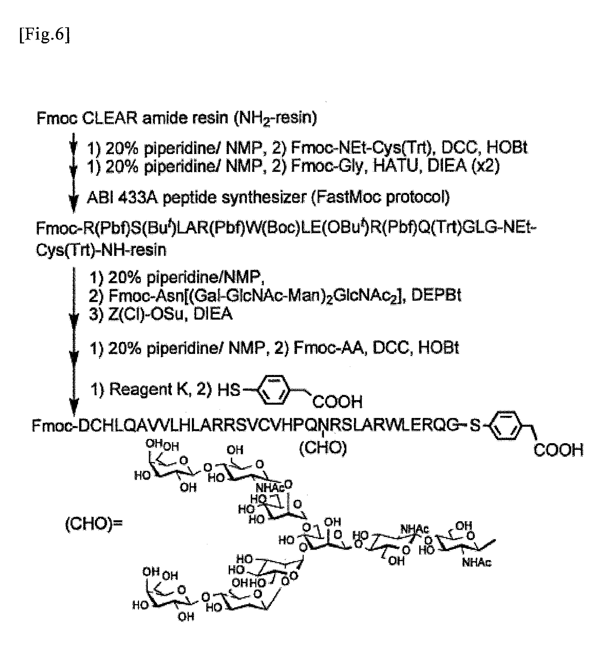

### Example 12-4: Example of synthesis of glycopeptide thioester [CCL27(37-69)] (Fig. 6) using amino acid having unprotected nonasaccharide

Fmoc-CLEAR amide resin (430 mg, 0.2 mmol) was treated with 20% piperidine/NMP for 5 minutes and the reaction was repeated with fresh reagent for 15 minutes. A solution prepared through 30 minutes of reaction of Fmoc-N-Et-Cys(Trt) (245 mg, 0.4 mmol), 1 M HOBt/NMP (0.5 ml), and 1 M DCC/NMP (0.5 ml) was added to the resin, followed by 1 hour of reaction. After washing with NMP, the resin was shaken in 10% Ac₂O-5% DIEA/NMP for 5 minutes. After washing with NMP, the resultant was treated with 20% piperidine/NMP for 5 minutes and the reaction was repeated with fresh reagent for 15 minutes. Fmoc-Gly (300 mg, 1.0 mmol), HATU (360 mg, 0.95 mmol), and DIEA (0.35 ml, 2.0 mmol) were added, followed by 1 hour of reaction at 50°C. The same amounts of Fmoc-Gly, HATU, and DIEA were added again, followed by 1 hour of reaction at 50°C. Peptide chain elongation was carried out on the thus obtained resin by the FastMoc method using a peptide synthesizer. Thus, Arg(Pbf)-Ser(Bu*^{t}*)-Leu-Ala-Arg(Pbf)-Trp(Boc)-Leu-Glu(OBu*^{t}*)-Arg(Pbf)-Gln(Trt)- Gly-N-Et-Cys(Trt)-NH-resin was obtained. A solution of Fmoc-Asn [(Gal-GlcNAc-Man)₂Man-GlcNAc₂] (57 mg, 29 µmol), DEPBT (17 mg, 57 µmol), and DIEA (20 µl, 104 µmol) dissolved in DMSO (0.4 ml) was added to a portion (19 µmol) of the thus obtained resin. The solution was shaken at 50°C for 3 hours and then washed with NMP. A solution of Z(Cl)-OSu (57 mg, 0.2 mmol) and DIEA (0.07 ml, 0.4 mmol) dissolved in NMP (0.4 ml) was added. The resultant was shaken at room temperature for 20 minutes. The remaining amino acid sequences were elongated by hand using an NMP solution (2.5 ml) of Fmoc-amino acid (0.1 mmol), DCC (0.15 mmol), and HOBt (0.15 mmol). Reagent K (3.5 ml) was added to the thus obtained.
Fmoc-Asp(OBu*^{t}*)-Cys(Acm)-His(Trt)-Leu-Gln(Trt)-Ala-Val-Val-Leu-His(Trt)-Leu-Al a-Arg(Pbf)-Arg(Pbf)-Ser-Val-Cys(Acm)-Val-His(Trt)-Pro-Gln(Trt)-Asn[(Gal-GlcNAc -Man)2Man-GlcNAc2]-Arg(Pbf)-Ser(Bu*^{t}*)-Leu-Ala-Arg(Pbf)-Trp(Boc)-Leu-Glu(OBu*^{t}*) -Arg(Pbf)-Gln(Trt)-Gly-N-Et-Cys(Trt)-NH-resin (0.17 g), followed by 2 hours of shaking at room temperature. TFA was removed by nitrogen stream and then ether was added, so as to cause precipitation. After 3 times of washing with ether, the precipitate was dried under reduced pressure. A crude peptide was dissolved in a 10% acetonitrile aqueous solution (30 ml) containing 2% mercaptophenyl acetic acid (MPAA), allowed to stand at room temperature for 2 days, and then purified by HPLC.
Thus, CCL27(37-69)thioester Fmoc-Asp-Cys(Acm)-His-Leu-Gln-Ala-Val-Val-Leu-His-Leu-Ala-Arg-Arg-Ser-Val-C ys(Acm)-Val-His-Pro-Gln-Asn[(Gal-GlcNAc-Man)2Man-GlcNAc2]-Arg-Ser-Leu-Ala-Arg-Trp-Leu-Glu-Arg-Gln-Gly-SC₆H₄CH₂COOH (3.4 mg, 0.57 µmol, 3.0%) was obtained.
MALDI-TOF MS: found: m/z 5989.6 (M+H)⁺, calcd for: (M+H)⁺ 5988.6.
Amino acid analysis: Asp_{0.95}Thr_{1.68}Ser_{2.81}Glu_{4.43}PrO_{3.99}Gly_{l.00}
Ala_{1.07}Val_{0.62}Met_{0.97}Ile_{0.52}Leu_{6.84}Tyr_{0.75}His_{0.90}Arg_{3.66}.

### Industrial Applicability

According to the present invention, a peptide thioester or an N-linked or O-linked glycopeptide thioester which is essential for chemical synthesis of proteins or glycoproteins can be synthesized with high yields via solid phase synthetic method. Proteins or glycoproteins that can be obtained by the method can be used for production of pharmaceutical products. Moreover, it is inferred that glycoproteins produced according to the present invention exert activity similar to that of naturally derived glycoproteins and specifically exert effects of accelerating the production of matrix metalloproteinase from fibroblasts.

### Brief Description of the Drawings

Fig. 1 shows the outline of the method for producing a peptide thioester according to the present invention.
Fig. 2 shows the method for producing an N-alkylcysteine derivative.
Fig. 3 shows the outline of peptide synthesis and thioesterification using Glu-Val-Thr-Gly-His-Arg-Trp-Leu-Lys-Gly as a model peptide.
Fig. 4 shows the course of the reaction when N-ethyl-cysteine was used. * denotes a peak derived from MPA.
Fig. 5 shows the synthetic route of emmprin (34-58) thioester having chitobiose (GlcNAc₂).
Fig. 6 shows the syntheteic route of CCL27 (37-69) thioester having nonasaccharide.

## Claims

1. A method for producing a peptide thioester, comprising the steps of:
(i) deprotecting Fmoc-NH-resin and then reacting the resultant with Fmoc-(amino acid residue)n-N(R)-CH(CH₂SY)-C(=O)OH (where n is 0 or 1) so as to produce a Fmoc-(amino acid residue)n-N(R)-CH(CH₂SY)-C(=O)NH-resin;
(ii) deprotecting the Fmoc group of the Fmoc-(amino acid residue)n-N(R)-CH(CH₂SY)-C(=O)NH-resin (where n is 0 or 1) obtained in step (i), and if necessary, reacting the resultant with Fmoc-amino acid, so as to produce Fmoc-(amino acid residue)n-N(R)-CH(CH₂SY)-C(=O)NH-resin (where n is 1 or 2), and then repeating Fmoc deprotection and reaction with Fmoc-amino acid, so as to produce X-N(R)-CH(CH₂SY)-C(=O)-NH-resin;
(iii) causing a deprotecting reagent to act on the X-N(R)-CH(CH₂SY)-C(=O)-NH-resin obtained in step (ii), so as to carry out release from the resin and deprotection of a thiol group; and
(iv) causing acidic thiol to act on the compound obtained in step (iii), so as to produce a thioester compound;
(where Fmoc denotes a 9-fluorenylmethoxycarbonyl group, R denotes an alkyl group having carbon number of 1 to 12 or an aralkyl group having carbon number of 7 to 12, Y denotes a protecting group for a thiol group, and X denotes a peptide residue).

2. The method according to claim 1, wherein the deprotecting reagent in step (iii) is trifluoroacetic acid.

3. The method according to claim 1 or 2, wherein the acidic thiol in step (iv) is mercaptocarboxylic acid or a mixture of mercaptan and carboxylic acid.

4. The method according to any one of claims 1 to 3, wherein the acidic thiol in step (iv) is HSCH₂CH₂COOH (MPA), HSC₆H₄CH₂COOH (MPAA), or a mixture of thio phenol and acetic acid.

5. The method according to any one of claims 1 to 4, wherein R is a methyl group, an ethyl group, an isobutyl group, or a benzyl group.

6. The method according to any one of claims 1 to 5, wherein Y is a trityl group.

7. A compound represented by Z-N(R)-CH(CH₂SY)-C(=0)OH (where Z denotes a hydrogen atom or a 9-fluorenylmethoxycarbonyl group, R denotes an alkyl group having carbon number of 1 to 12 or an aralkyl group having carbon number of 7 to 12, and Y denotes a protecting group for thiol).

8. A method for producing the compound according to claim 7, comprising the steps of:
(i) reacting a compound represented by YSCH₂CH(NH₂)C(=O)OH (where Y is a protecting group for a thiol group) with a compound represented by R¹CHO (where R¹ denotes a hydrogen atom, a C1-11 alkyl group, or a C6-11 aryl group), so as to produce a compound reprsented by YSCH₂CH(N=CHR¹)C(=O)OH (where Y and R¹ are as defined above); and
(ii) causing a hydrogenating agent to act on the compound represented by YSCH₂CH(N=CHR¹)C(=O)OH (where Y and R¹ are as defined above) obtained in step (i), so as to produce a compound represented by YSCH₂CH(NHCH₂R¹)C(=O)OH (where Y and R¹ are as defined above), and, if desired, causing 9-fluorenylmethoxycarbonyl-N-hydroxysuccinimide ester to act, so as to protect the amino group with the 9-fluorenylmethoxycarbonyl group.

9. The method according to claim 8, wherein the hydrogenating agent in step (ii) is NaBH₄ or NaBH₃CN.
